# EUROPÄISCHE PATENTANMELDUNG

(11) **EP 1 075 847 A1**
(43) Veröffentlichungstag der Anmeldung: **14.02.2001**
(21) Anmeldenummer: 99250154.4
(22) Anmeldetag: 13.08.1999
(51) Int. Cl.: A61M 5/20

(54) **Autoinjektion zur Vermeidung eines Herzinfarkts**

(71) Anmelder: Strunz, Walter A., 22395 Hamburg (DE)
(72) Erfinder: Strunz, Walter A., 22395 Hamburg (DE)

(57) **Zusammenfassung**

Die Anmeldung betrifft ein Verfahren zur Ausschaltung des tödlichen Risikos bei Infarktgefährdung.

Das Verfahren ist dadurch gekennzeichnet, daß sich der Betroffene durch Eigeninitiative eine Autoinjektion verabreicht.

## Beschreibung

### 1. Ersuchen an das EUROPÄISCHE PATENTAMT BERLIN

Mit folgender Darstellung Überlebenswichtiger Tatsachen ersuche ich das EUROPÄISCHE PATENTAMT IN BERLIN um Erteilung eines Patents.

### 2. Technik und Humanität

Die Erfindung
* ANT I NFARKT *
ist sowohl konventioneller technischer Natur als auch eine humanitäre Großtat. Sie rettet auf direktestem Wege menschenleben, RETTET vor dem TOD durch Herzinfarkt.

### 3. Tödliches Risiko sowie Eigeninitiative

Tatsächlich schaltet
* ANT I NFARKT *
Das tödliche Risiko bei Infarktgefährdung aus, indem es der Person, welche bis zum heutigen Tage in wenigen Minuten an einem Herzinfarkt stirbt, die Eigeninitiative in die Hand gibt.

### 4. Selbstbestimmtes Handeln

Der Betroffene verabreicht sich die
Selbst (Auto) Injektion
und löst damit jene physiologischen Prozesse aus, welche die Probleme durch Blutgerinsel, Verkrampfungen dort erleichtern, wo sonst die tödlichen Folgen eintreten.

### 5. Empirisch wissenschaftliche Untersuchung

Aktuell, am 18. März 1999, meldete die Frankfurter Allgemeine Zeitung in einem dpa - Artikel Daten von 15.000 akuten Herz-infark Fällen. 230 deutsche Kliniken haben daran mitgearbeitet. Jedes Jahr endet der akute Herzinfarkt für 200.000 Menschen tödlich. Anlage Kopie.

### 6. Zuversicht in die eigene Kraft

* ANT I NFARKT *
gibt den Betroffenen die Zuversicht in die Leistungsfähigkeit des eigenen Organismus, den Herzinfarkt lebendig zu überstehen.

### 7. Überlebenswille und * ANT I NFARKT *

Der eigene Wille, die eigene Überzeugung des infarktgefährdeten Menschen zusammen mit der
Selbst (Auto) Injektion
eröffnet die Chance, die tödlich kritische Situation zu meistern.

### 8. Nachfragepotential

Im europäischen Raum besteht ein durch Bevölkerungsstatistiken belegtes Nachfragepotential:
5 0 Millionen Menschen
wollen Vorsorge für den Ernstfall treffen, daß sie an einem Herzinfarkt zu sterben drohen.

### 9. Umsatzvolumen

Bei einem Apothekenabgabepreis von DM 45,-- pro Artikel/ * ANT I NFRARKT * errechnet sich ein Umsatzvolumen in Höhe von
2 , 2 5 Milliarden DM

### 10. Gewerbliche Nutzung

Mit diesem vorsichtig prognostizierten Umsatzvolumen sind die gewerbliche Nutzung der Erfindung * ANT I NFARKT * bewiesen und die Voraussetzungen für die Patentierung erfüllt.

## Patentansprüche

1. Mein Patentanspruch ist dadurch gekennzeichnet, daß der Herzinfarktpatient selbst die Injektion vornimmt und damit jene physiologischen Prozesse auslöst, welche die Probleme durch Blutgerinsel, Verkrampfungen dort erleichtern, wo sonst die tödlichen Folgen eintreten.

2. In der Hauptsache ist mein Patentanspruch dadurch gekennzeichnet, daß erstmalig die kausale Identität: Tödliche Infarktgefährdung und Rettung durch Selbst (Auto) Injektion
* ANT I NFARKT *
als humanitäre Großtat den Betroffenen das Leben rettet.
